# EUROPÄISCHE PATENTSCHRIFT

(11) **EP 1 319 062 B1**
(45) Veröffentlichungstag und Bekanntmachung des Hinweises auf die Patenterteilung: **12.03.2008**
(21) Anmeldenummer: 01969578.2
(22) Anmeldetag: 08.08.2001
(51) Int. Cl.: C12M 1/20, B01L 3/00

(54) **VERFAHREN UND VORRICHTUNG ZUM ZÜCHTEN UND/ODER BEHANDELN VON ZELLEN**
METHOD AND DEVICE FOR GROWING AND/OR TREATING CELLS
PROCEDE ET DISPOSITIF POUR CULTIVER ET/OU TRAITER DES CELLULES

(30) Priorität: 19.09.2000 DE 10046175
(43) Veröffentlichungstag der Anmeldung: 18.06.2003
(73) Patentinhaber: Bader, Augustinus, 31275 Lehrte-Immensen (DE)
(72) Erfinder: Bader, Augustinus, 31275 Lehrte-Immensen (DE)
(74) Vertreter: Benz, Jürgen
(86) Internationale Anmeldenummer: PCT/EP2001/009159
(87) Internationale Veröffentlichungsnummer: WO 2002/024861

(56) Entgegenhaltungen:
- WO-A-02/11880
- WO-A-97/33179
- DE-U- 29 519 602
- GB-A- 2 269 391
- US-A- 5 462 874
- US-A- 5 707 869

## Beschreibung

Die Erfindung betrifft ein Verfahren zum automatisierten Züchten und/oder Behandeln von Zellen für diagnostische Zwecke.

In der älteren DE 199 35 643.2 A1 ist eine Vorrichtung zum Züchten und/oder Behandeln von Zellen beschrieben, wobei auf einem Träger ein formbarer Zellkulturraum angeordnet ist. Der Zellkulturraum wird dabei durch den Träger oder eine Trägerfolie auf einer Seite und eine Zellkulturfolie auf der anderen Seite, die elastisch ist, gebildet. Mit einer Vorrichtung dieser Art, ist es möglich, eine Massenkultur von Zellen mit großer Variabilität und für viele Einsatzzwecke durchzuführen.

Die WO 97/33179 beschreibt ein Verfahren zum Testen von chemischen Stoffen auf ihre Wirksamkeit als Pflanzenschutzmittel unter Einsatz einer zahlreiche Bohrungen aufweisenden Kulturplatte, in welche Sprüheinrichtungen sowie Zu- und Rücklaufbohrungen eingebracht werden. Das System erlaubt das Testen von Stoffen und Pflanzen im vollautomatischen Betrieb.

Das US Patent 5,707,869 beschreibt eine Zellkulturvorrichtung, welche eine Wanne, einen Deckel und eine Multiwellplatte aufweist, wobei jede der Öffnungen mir einer unteren gaspermeablen Folie für die Zufuhr von Sauerstoff und einer semipermeablen Folie versehen ist.

Auch die US 5,462,874 lehrt eine entsprechende Zellkulturvorrichtung, die eine in eine Wanne eingebrachte Multiwellplatte umfasst, wobei die Vertiefungen am oberen Ende offen und am unteren Ende mittels einer semipermeablen Folie verschlossen sind.

Eine Vorrichtung ähnlicher Bauweise und Verwendung wird in dem deutschen Gebrauchsmuster DE 2 95 519 602 beschrieben. Hierbei enthalten die Vertiefungen neben der Probe eine Nährlösung. Zu- und Rücklaufleitungen zur Sauerstoffversorgung werden mittels Einsätze in besagte Vertiefungen eingebracht.

Die GB 2269391 offenbart ein Verfahren zur Züchtung von Mikroorganismen mittels einer Mikrowellplatte, wobei jede Vertiefung mit einer semipermeablen Folie verschlossen ist, um zu verhindern, das Nährmedium austreten kann, aber die Sauerstoffversorgung der Mikroorganismen durch Diffusion ermöglicht.

Zum weiteren Stand der Technik wird auch auf die DE 197 19 751 A1 verwiesen.

Der vorliegenden Erfindung liegt die Aufgabe zugrunde, ein Verfahren zum automatisierten Züchten und/oder Behandeln von Zellen zu schaffen, wobei auf möglichst geringem Raum eine hohe Anzahl von Zellen, die auch von unterschiedlicher Art sein können, für diagnostische Zwecke gezüchtet und/oder behandelt werden können.

Erfindungsgemäß wird diese Aufgabe durch das in Anspruch 1 genannte Verfahren gelöst.

Erfindungsgemäß wird dynamisches Verfahren unter Verwendung einer Zellkulturplatte eingesetzt. Dies bedeutet, durch die Zufuhr von Sauerstoff und/oder Nährstoffen, welche kontinuierlich oder auch diskontinuierlich erfolgt, lassen sich nunmehr Zellen über einen längeren Zeitraum züchten und/oder behandeln und beobachten. Die hierzu erforderliche Zufuhr von Nährstoffen und Sauerstoff kann auf die verschiedenartigste Weise erfolgen.

In einfacher Weise können dabei Nährstoffe und Sauerstoff gemeinsam durch eine kontinuierliche oder auch diskontinuierliche Durchströmung der nunmehr zu Zellkulturräumen umfunktionierten Vertiefungen bzw. Bohrungen geleitet werden.

In einer sehr vorteilhaften Ausgestaltung der Erfindung kann jedoch eine separate Zufuhr von Sauerstoff in den Zellkulturraum von der Unterseite der Zellkulturplatte aus über eine gaspermeable Folie oder Membrane eingebracht werden. Normalerweise sind die Zellkulturplatten mit stabilen, gasundurchlässigen Böden versehen. Ersetzt man nun diese Böden durch eine entsprechende gaspermeable Folie bzw. Membrane und sorgt für eine entsprechende Sauerstoff- bzw. Luftzufuhr zu diesen Bereichen, so ist eine einfache und sehr intensive Sauerstoffzufuhr zu den Zellen gegeben.

Eine weitere Möglichkeit zur Zufuhr von Nährstoffen und/oder Sauerstoff kann darin bestehen, daß in wenigstens einen Teil der Bohrungen bzw. Zellkulturräume der Zellkulturplatte Einsätze einsetzbar sind, welche jeweils mit Zulaufbohrungen und Rücklaufbohrungen versehen sind.

Vorteilhafte Ausgestaltungen und Weiterbildungen der Erfindung ergeben sich aus den übrigen Unteransprüchen und aus den nachfolgend anhand der Zeichnung beschriebenen Ausführungsbeispielen.

Es zeigt:
- Fig. 1: eine Zellkulturplatte (Multiwellplatte) in perspektivischer Ansicht;
- Fig. 2: einen Schnitt nach der Linie II-II der Fig. 1 in vergrößerter Darstellung mit der Ausbildung der Bohrungen der Zellkulturplatte, jeweils als Zellkulturraum;
- Fig. 3: einen Schnitt nach der Linie III-III der Fig. 2;
- Fig. 4: eine alternative Ausgestaltung der Ausbildung von Zellkulturräumen in einem Schnitt ähnlich dem nach der Fig. 2;
- Fig. 5: eine weitere Ausgestaltung zur Bildung von Zellkulturräumen, ebenfalls in einem Schnitt entsprechend dem nach der Fig. 2;
- Fig. 6: prinzipmäßig die Zufuhr und Abfuhr von Nährstoffen zu den Zellkulturräumen in einer Draufsicht; und
- Fig. 7: ausschnittsweise eine Ausgestaltung, wobei die Zellkulturräume unter Druck setzbar sind.

Zum Züchten und/oder Behandeln von Zellen für diagnostische Zwecke wird eine an sich bekannte Zellkulturplatte 1 (Multiwellplatte) verwendet, die im allgemeinen entsprechend ihrer Größe, eine hohe Anzahl von Löchern bzw. Bohrungen 2 aufweist. Die Bohrungen 2 haben Durchmesser von wenigen Millimetern bis zu mehreren Millimetern, und deren Anzahl kann bis zu mehreren Hundert Stück pro Zellkulturplatte 1 betragen.

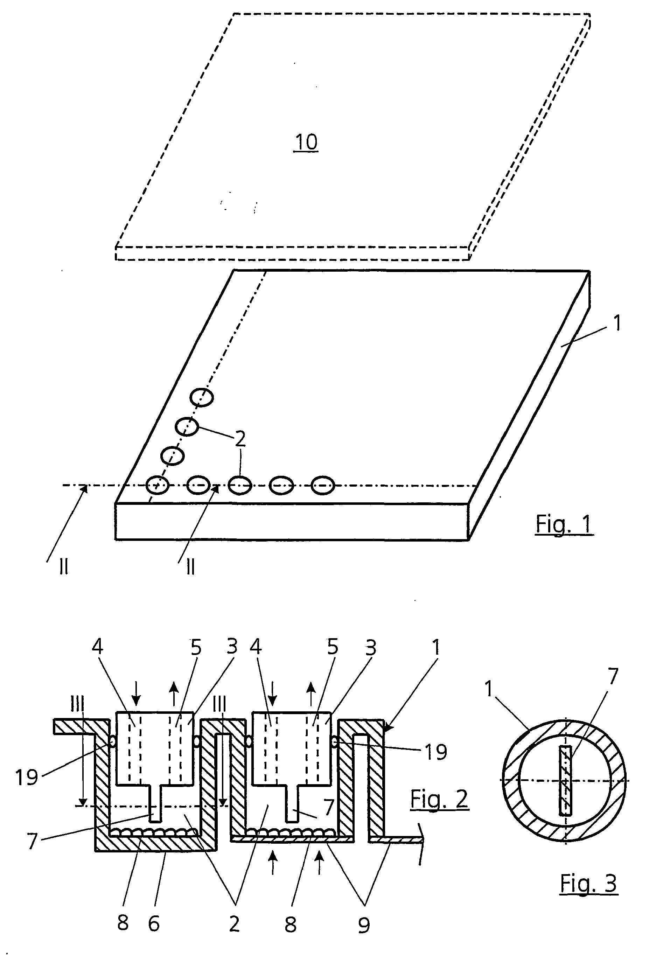

Aus den Figuren 2 bis 5 sind verschiedene Ausgestaltungen der Bohrungen 2 bzw. deren Umfunktionierung zu Zellkulturräumen 2' ersichtlich. Gemäß Fig. 2 wird in jede Bohrung 2, die als Zellkulturraum 2' dienen soll, ein Einsatz 3 eingesetzt, der mit einer Zulaufbohrung 4 und einer Rücklaufbohrung 5 versehen ist, welche sich von der Oberseite der Zellkulturplatte 1 bis zu deren Unterseite erstrecken, wobei die Unterseiten der Zellkulturplatte bzw. die Bohrungen 2 mit Böden 6 abgeschlossen sind. Die Einsätze 3 werden somit von der Oberseite der Zellkulturplatte 1 aus in die offenen Bohrungen 2 eingesetzt. Zur Abdichtung der Zellkulturräume 2' sind die Einsätze 3 jeweils umfangseitig mit einem Dichtring 19 versehen.

Von der jeweils zu dem Boden 6 gerichteten Seite eines Einsatzes 3 aus, ragt ein Trennsteg 7 in Richtung auf den Boden 6. Der Trennsteg 7 liegt zwischen der Zulaufbohrung 4 und der Rücklaufbohrung 5 und erstreckt sich, wie aus der Fig. 3 ersichtlich ist, quer über die Bohrung 2. Durch den Trennsteg 7 wird erreicht, daß die über die Zulaufbohrung 4 eingebrachten Nährstoffe nicht in einem "Kurzschluß" direkt zu der Rücklaufbohrung 3 strömen können. Durch den Trennsteg 7 werden sie gezwungen, entsprechend den gesamten Zellkulturraum 2' zu durchströmen und dabei die auf den Boden 6 aufgebrachten Zellen 8 zu überströmen. Gleichzeitig kann der Trennsteg 7 auch zur Zentrierung bzw. zum leichteren Einführen des Einsatzes 3 in die Bohrung 2 dienen.

Bei der in der Fig. 2 links dargestellten Ausgestaltung mit dem Einsatz 3 erfolgt die Sauerstoffzufuhr zu den Zellen 8 zusammen mit der Zufuhr von Nährstoffen über die Zulaufbohrung 4. Entfernt man den festen Boden 6 und ersetzt diesen durch eine gaspermeable Folie oder Membrane 9 entsprechend der rechts in der Figur in der Dicke stark vergrößert dargestellten Ausgestaltung, so kann eine sehr intensive und direkte Sauerstoffzufuhr von der Unterseite der Zellkulturplatte 1 aus durch die gaspermeable Folie erfolgen (siehe Pfeile). Auf der gäspermeablen Folie 9, die in den Figuren aus Übersichtlichkeitsgründen wesentlich dicker dargestellt ist, erfolgt die Zucht und die Behandlung der Zellen 8.

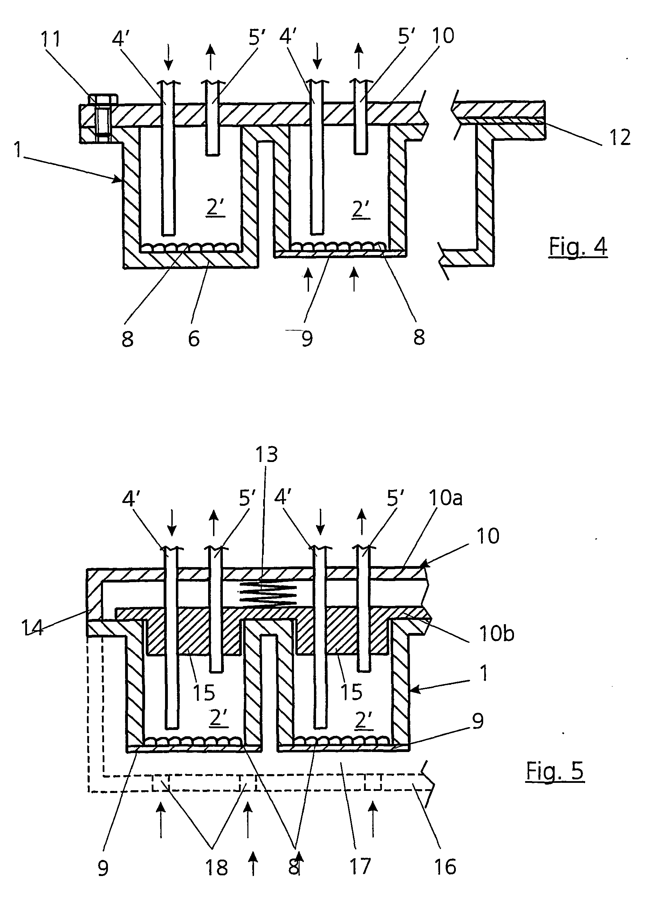

Die Fig. 4 zeigt anstelle einer Vielzahl von in die Bohrungen 2 einzusetzenden Einsätze 3 eine Deckplatte 10, welche in der Fig. 1 nur ganz allgemein gestrichelt dargestellt ist. In die Deckplatte 10 sind in entsprechenden Bohrungen der Deckplatte Zulaufanschlüsse 4' und Rücklaufanschlüsse 5' eingesetzt. Wie ersichtlich, ragen die Zulaufanschlüsse 4' im Vergleich zu den Rücklaufanschlüssen 5' deutlich tiefer in die dazugehörigen Bohrungen 2 bzw. die Zellkulturräume 2'. Wie weiter ersichtlich, wird durch diese Maßnahme ebenfalls erreicht, daß keine Kurzschlußströmung direkt von einem Zulauf zu einem Rücklauf erfolgt. Die Zellen 8 werden vielmehr von dem Nährmedium überströmt. In der Fig. 4 sind ebenfalls die beiden Varianten mit einem festen Boden 6 und einer gaspermeablen Folie bzw. Membrane 9 nebeneinander dargestellt.

Links in der Fig. 4 ist dargestellt, wie die Deckplatte 10 durch Schrauben 11 mit der Zellkulturplatte 1 verbunden ist. Rechts ist dargestellt, daß anstelle oder zusätzlich zu einer Verbindung über Schrauben 11 auch eine Abdichtung durch eine Dichtungseinrichtung 12 zwischen der Deckplatte 10 und der Zellkulturplatte 1 erreicht werden kann.

In der Fig. 5 ist prinzipmäßig eine zweiteilige Ausbildung der Deckplatte 10 mit einer oberen Abdeckung 10a und einer unteren Abdeckung 10b dargestellt. Wie ersichtlich, sind die obere Abdeckung 10a und die untere Abdeckung 10b abstandseinstellbar miteinander verbunden. Durch dazwischen gelegte Federn 13 wird eine Vorspannung erreicht. Die obere Abdekkung 10a wird über Stege 14 an den Rändern der Zellkulturplatte 1 umlaufend auf diese aufgesetzt. Die untere Abdekkung 10b ist mit Bohrungen versehen, durch die Zulaufanschlüsse 4' und Rücklaufanschlüsse 5', ähnlich der Ausgestaltung nach der Fig. 4, geschoben sind. Die Zulaufanschlüsse 4' und Rücklaufanschlüsse 5' sind entsprechend auch durch die obere Abdeckung 10a entweder auf deren Oberseite oder gegebenenfalls auch seitlich an den Rändern herauszuführen. Durch die Federn 13 wird eine Abdichtung der Zellkulturräume erreicht, weil die untere Abdeckung 10b entsprechend dicht auf der Oberseite der Zellkulturplatte 1 aufliegt. Gleichzeitig werden auf diese Weise auch Ansätze 15, die auf der Unterseite der unteren Abdeckung 10b nach unten aus dieser vorstehen in die Bohrungen 2 zur Bildung von abgeschlossenen Zellkulturräumen 2' eingeführt.

Aus der Fig. 5 ist in gestrichelter Darstellung auch ersichtlich, daß man die gesamte Einheit mit der Zellkulturplatte 1 in eine Wanne 16 setzen kann. Die Zellkulturplatte 1 sitzt dabei umlaufend dicht auf der Wanne 16 auf. Zwischen den in diesem Falle aus einer gaspermeablen Folie bzw. Membrane 9 gebildeten Boden und dem Wannenboden befindet sich ein Zwischenraum 17. Führt man Sauerstoff in die Wanne 16 ein, z.B. über Bohrungen 18, so erfolgt eine sehr intensive direkte Sauerstoffversorgung für die in den Zellkulturräumen 2' liegenden Zellen 8 durch die Folie bzw. Membrane 9.

Die Bohrungen 18 können vorzugsweise der Größe der Bohrungen 2 entsprechen. Der Zwischenraum 17 kann gegebenenfalls entfallen, wobei in diesem Fall die Zellkulturplatte 1 direkt auf dem Boden der Wanne 16 aufsitzt und über eine, vorzugsweise dem Durchmesser der Zellkulturräume 2' entsprechende Öffnung, mittels einer gaspermeablen Folie 9 bzw. Membran 9 ein Gasaustausch erfolgen kann. Die Bohrungen 18 in der Wanne 16 ermöglichen noch eine Sichtverbesserung bei mikroskopischer Beobachtung der Zellen. Vorzugsweise ist hierfür auch das Material der Deckplatte 10 wie auch der Wanne 16 aus transparentem Material hergestellt, wie z.B. Polycarbonat oder Polysteren.

Mit dem erfindungsgemäßen Verfahren wird aus einer an sich bekannten Zellkulturplatte 1 eine Art Bioreaktor zum Züchten und/oder Behandeln von Zellen. Durch die dynamische Zufuhr von Nährmedium und Sauerstoff ist dabei auch eine Langzeitkultur möglich.

Ein weiterer Vorteil der Erfindung ist die Automatisierungsmöglichkeit von routinemäßig ablaufenden Prozessen, wie z.B. von in beliebigen nach oben offenen Kulturgefäßen.

An sich bekannte Zellkulturplatten herkömmlicher Bauart können auf einfache Weise auch für das erfindungsgemäße Verfahren abgewandelt werden. So kann z.B. auf einfache Weise der feste Boden 8 entfernt werden und eine gaspermeable Folie 9 über die nun auf beiden Seiten offenen Bohrungen 2 auf der Unterseite der Zellkulturplatte 1 gespannt werden. Über die gaspermeable Folie 9 kann man die Entwicklung der Zellen 8 auch im Bedarfsfall beobachten, wozu die Folie 9 entsprechend durchsichtig auszubilden ist.

Dadurch, daß die Zellen 8 sehr definiert als Schicht mit zweidimensionaler Ausdehnung in den Zellkulturräumen 2' wachsen bzw. angeordnet sind, läßt sich die gesamte Zellkulturplatte 1 im Bedarfsfalle auch einfrieren, wenn man die Zellen transportieren oder bis zu deren Verwendung zwischenlagern möchte.

In der Regel wird man für das Kulturverfahren alle oder einen Großteil der Bohrungen 2 einer Zellkulturplatte zu Zellkulturräumen 2' umfunktionieren. Im Bedarfsfalle ist es selbstverständlich auch möglich, auch nur einzelne Zellkulturräume 2' mit entsprechend einzelnen Einsätzen 3 zu schaffen, da die Einsätze 3 auch alleine einsetzbar sind. Die Einsätze 3 können auch bei einzelnen Kultureinheiten individuell angeströmt bzw. entleert werden.

Wesentlich ist in jedem Falle, daß für eine ausreichende Sauerstoffversorgung zu den Zellen 8 gesorgt wird, um auch ein länger dauerndes Zellkulturverfahren, insbesondere für "anspruchsvolle" Zellen, durchführen zu können. Aufgrund der Ausgestaltung der bekannten Zellkulturplatten 1, insbesondere der Bohrungen 2, war eine Züchtung und/oder Behandlung von Zellen in der erfindungsgemäßen Form bisher nicht möglich.

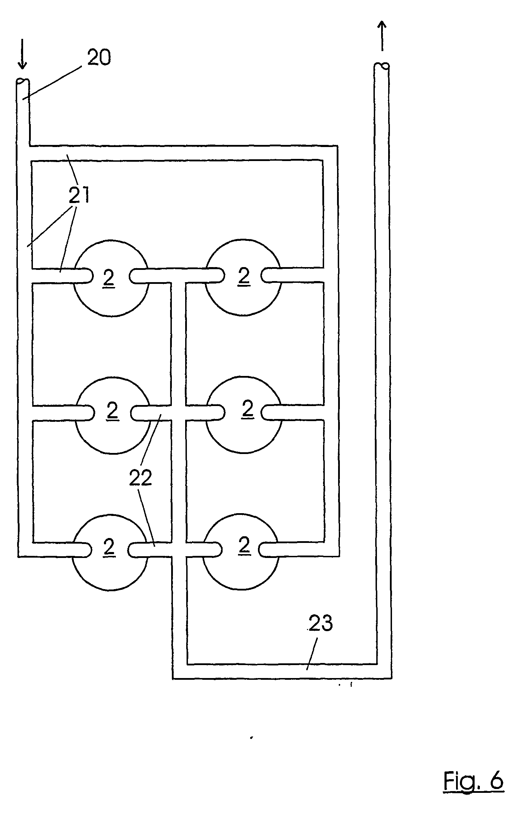

In der Fig. 6 ist prinzipmäßig die Zufuhr und die Abfuhr von Nährstoffen und gegebenenfalls von Sauerstoff zu den Zellkulturräumen 2 in einer Draufsicht dargestellt. Wie ersichtlich, ist dabei ein gemeinsamer Zulaufanschluß 20 vorgesehen, von welchem aus über mehrere Zweigleitungen 21 die einzelnen Zellkulturräume 2 mit Nährmedium und gegebenenfalls auch mit Sauerstoff versorgt werden. Anschließend folgt wiederum über einzelne Zweigleitungen 22 eine gemeinsame Abfuhr über einen Ablaufanschluß 23.

Durch den gemeinsamen Zulaufanschluß 20 und den gemeinsamen Ablaufanschluß 23 läßt sich die Vorrichtung z.B. in Form einer "docking station" verwenden, die man an eine Zentralstation anschließt, eventuell gemeinsam mit anderen Vorrichtungen. Auf diese Weise lassen sich sehr große Brutstöcke bzw. Zuchtvorrichtungen schaffen.

Die gemeinsame Zufuhr und die gemeinsame Abfuhr mit den Zweigleitungen 21 und 22 kann z.B. durch entsprechende Leitungen, Aussparungen oder Kanäle in der Deckplatte 10 erfolgen, von wo aus dann über einzelne Zweigleitungen 21 jeweils einzeln die Zellkulturräume 2 versorgt werden. In Verbindung mit der Wanne 16 läßt sich auf diese Weise eine Art Kassettenstruktur schaffen. Im Bedarfsfalle können auch mehrere Einheiten übereinander angeordnet werden. Dies ist z.B. dadurch möglich, daß man mehrere Zellkulturplatten 1 übereinander anordnet, wobei die Versorgung mit Nährmedium und mit Sauerstoff durch dazwischenliegende Deckplatten 10 erfolgen kann, welche entsprechend, z.B. jeweils auf ihrer Oberseite und auf ihrer Unterseite, mit gemeinsamen Zulaufenanschlüssen 20, Zweigleitungen 21 und 22 und gemeinsamen Ablaufanschlüssen 23 versehen sind.

Je nach Größe der Zellkulturräume 2 und deren Anzahl kann es gegebenenfalls erforderlich sein, daß mehrere Deckplatten 10 mit darin angeordneten Zulaufanschlüssen 20, Zweigleitungen 21 und 22 und Ablaufanschlüssen 23 vorgesehen sind, die aus Platzgründen mit entsprechend versetzt angeordneten Zweigleitungen 21 und 22 versehen sind, damit alle Zellkulturräume 2 versorgt werden können.

Zusätzlich und alternativ kann man auch die Zellkulturplatten 1 selbst mit gemeinsamen Zulaufanschlüssen 20, Zweigleitungen 21 und 22 und mit gemeinsamen Ablaufanschlüssen 23 jeweils versehen, um jeden Zellkulturraum 2 erreichen zu können. Hierzu kann man z.B. eine Zellkulturplatte 1 in der Mitte aufteilen und im Bereich der Trennebene die Strömungskanäle einbringen. In jedem Fall sollte jedoch eine Kanalführung geschaffen werden, durch die eine gleichmäßige Versorgung aller Zellkulturräume 2 gewährleistet wird.

Die Folie bzw. die Membrane 9 kann im Bedarfsfalle auch mikroporös ausgestaltet sein, was den Vorteil hat, daß man nicht nur von oben, sondern zusätzlich oder alternativ auch von unten her Nährmedium in die Zellkulturräume 2' einbringen kann.

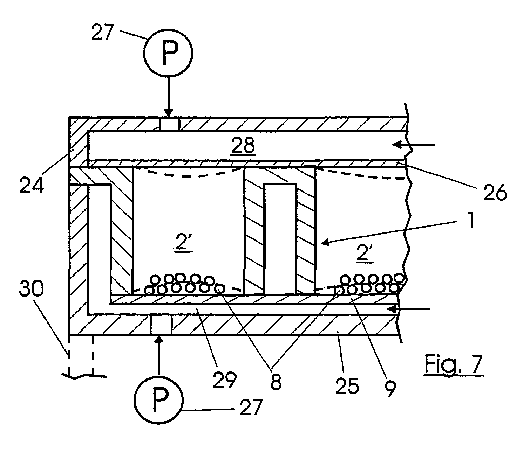

In der Fig. 7 ist eine sehr vorteilhafte Ausgestaltung dargestellt, wobei die Zellkulturräume 2' unter Druck gesetzt werden können. Hierzu ist die Zellkulturplatte 1 auf der Oberseite durch einen druckdichten Dom 24 und auf der Unterseite durch einen druckdichten Behälter 25 abgeschlossen. Die Öffnungen 2 auf der Oberseite der Zellkulturplatte 1 sind mit einer flexiblen Folie 26 abgeschlossen, welche im Bedarfsfalle auch gaspermeabel oder mikroporös sein kann, um von dieser Seite aus eine Versorgung der in den Zellkulturräumen 2' sich befindenden Zellen 8 mit Sauerstoff/Luft und/oder Nährmedium zu erreichen. Auf der Unterseite sind die Zellkulturräume 2' ebenfalls durch die Membrane 9, welche gaspermeabel oder mikroporös ausgebildet ist, abgeschlossen.

Durch nicht näher dargestellte Druckanschlüsse 27 kann über entsprechende Druckquellen ein Raum 28 zwischen dem Dom 24 und der Folie 26 und ein Raum 29 zwischen der Membrane 9 und dem Boden des Behälters 25 unter Über- oder Unterdruck gesetzt werden. Die Steuerung der Druckverhältnisse ist dabei beliebig. Dies bedeutet, es kann abwechselnd Druck von oben oder von unten oder auch gleichzeitig auf die Zellkulturräume 2' ausgeübt werden. Hierzu verformen sich die Folie 26 und die Membrane 9 entsprechend (siehe gestrichelte Darstellung). Auf diese Weise werden die Zellen 8 entsprechend mechanisch bewegt, was z.B. erhebliche Vorteile für die Erstellung von Geweben, wie z.B. Knochen, Knorpel, Muskulatur und dergleichen, besitzt, da durch Dehnungen im Sinne von Konditionierungen bzw. einem Training simuliert werden können. Zusätzlich können in den Zellkulturräumen 2' rhythmische oder intermittierende Druckbelastungen erzeugt werden. Durch diese Maßnahmen lassen sich in vivo-Verhältnisse besser simulieren.

Durch die Verwendung der Folie 26 sind die Zellkulturräume 2' im Bedarfsfalle auch Pipettierungsprozeßen zugänglich.

Anstelle einer einfachen Folie 26 können die Öffnungen 2 auch durch eine septumartiges Kunststoffmembran abgedeckt werden.

Anstelle von Druckbelastungen oder auch zusätzlich können elektrische Ströme den Zellen 8 auferlegt werden. Auf diese Weise können z.B. auch Dehnungen über elektromagnetische Felder ausgelöst werden, was z.B. für Herzmuskelzellen, ZNS-Zellen (neuronale Zellen) von Vorteil ist. Auf diese Weise kann man z.B. Medikamente testen, die die Herzfrequenz beschleunigen oder reduzieren, wozu die elektrischen Ströme praktisch Herzmuskelzellen elektrisch stimulieren. Auf diese Weise kommt es zu Interaktionen zwischen einem technischen und einem biologischen System.

Die vorstehend beschriebene Zellkultursysteme können im Bedarfsfalle auch in Form eines Bioreaktors bzw. im Sandwich-System miteinander kombiniert werden. Hierzu ist es lediglich erforderlich, anstelle einer Wanne 16 oder dem Behälter 25 einen entsprechend gestalteten Zwischenboden vorzusehen, so daß sich - wie in der Fig. 7 gestrichelt angedeutet - eine weitere Einheit 30 unter dem Behälter 25 bzw. unter einem entsprechenden Zwischenboden anschließt. Dabei können mehrere derartige Einheiten übereinander angeordnet werden. Der dabei entstehende Bioreaktor kann in diesem Falle stufenförmig aufgebaut sein oder auch spiegelbildlich, wobei sich an die in der Fig. 7 beschriebene Einheit spiegelbildlich eine weitere Einheit anschließt. In diesem Falle dient der Raum 29 zur Zufuhr von Luft/Sauerstoff und/oder Nährmedium sowohl für die Zellkulturplatte 1 als auch für die in der Einheit 30 sich befindende Zellkulturplatte (nicht dargestellt). Selbstverständlich sind im Bedarfsfalle jedoch auch getrennte Zu- und Abflüsse möglich.

Anstelle von einfachen Zellen 8 in den Zellkulturräumen 2' können selbstverständlich auch mehrschichtige Kulturen gezüchtet werden.

Als eines der wesentlichen Unterschiede zum Stand der Technik ist festzuhalten, daß es sich bei dem vorliegenden Zellkultursystem um dynamische Prozesse handelt, welche kontinuierlich oder intermittierend ablaufen können und nicht nur um eine statische Züchtung von Zellkultursystemen, welche nach Anlegen nicht mehr weiterbehandelt werden.

Grundsätzlich liegen bei dem vorliegenden System drei Verfahren mit dazugehörigen Vorrichtungen vor, nämlich:
1. Im einfachsten Fall sind geschlossene Böden vorhanden und die Versorgung der Zellkulturräume 2' erfolgt von oben über die Öffnungen bzw. Bohrungen 2 mit entsprechenden Zulaufbohrungen 4 und Rücklaufbohrungen 5.
2. Eine Ausgestaltung der Zellkulturplatten 1 mit einer gaspermeablen oder mikroporösen Membrane 9 auf der Unterseite und abgeschlossenen Bohrungen 2 auf der Oberseite, mit oder ohne Zulaufbohrungen 4 und Rücklaufbohrungen 5.
3. Elastische Folien sowohl auf der Oberseite als auch auf der Unterseite der Zellkulturplatte 1, die gaspermeabel oder mikroporös sein können und welche mit entsprechenden Drücken belastet werden. Die gaspermeable oder mikroporöse Membrane 9 auf der Unterseite kann aus einer Vielzahl von einzelnen Membranen gebildet sein, die die Zellkulturräume 2' auf der Unterseite jeweils abdecken. Im allgemeinen wird man jedoch eine Folie verwenden, die die gesamte Unterseite der Zellkulturplatte 1 entsprechend abdeckt, wie dies in der Fig. 7 dargestellt ist.

Erfindungsgemäß können nunmehr komplexe Zellkultursysteme, die entsprechend höhere Anforderungen an die Mikroumgebung stellen, behandelt werden. Dabei kann man in den Bohrungen 2 der Zellkulturplatte 1 auch eine 3-D Struktur mit zweidimensionaler Ausdehnung definieren, die in vivo dem Abstand von einer Kapillare zur nächsten Kapillare entspricht. Dieser bioartifizielle Gewebeschnitt dient der Erstellung organotypischer Kulturbedingungen auf kleinstem Raum und ermöglicht es somit auch komplexere Kokultursysteme unter Verwendung von verschiedenen Zelltypen und extrazellulärer Matrix dem High-Throughput-Screening zugänglich zu machen.

Grundsätzlich liegt bei dem erfindungsgemäßen Verfahren ein Dünnschichtkultursystem vor, das in vorteilhafter Weise von unten oxigeniert wird und das der physiologischen Zelldichte entspricht, und zwar in dem Abstand einer Blutkapillare im Organismus bis zur nächsten Blutkapillare.

Eines der Hauptvorteile der vorliegenden Erfindung liegt in der Miniaturisierung mit möglichst kleinen Einheiten auf engem Raum. Auf diese Weise werden auch wenig Zellen für ein Kulturverfahren benötigt, die sich dann während des Verfahrens entsprechend vermehren können.

Eines der Haupteinsatzgebiete des erfindungsgemäßen Verfahrens hierzu ist deshalb die Untersuchung bzw. die Auswirkung von Chemikalien und Pharmazeutikas auf Zellen, insbesondere auf menschliche Zellen. Auf diese Weise können Tierversuche, die sehr aufwendig und teuer durchzuführen sind, zumindest teilweise ersetzt werden.

## Patentansprüche

1. Verfahren zum automatisierten Züchten und/oder Behandeln von Zellen für diagnostische Zwecke mittels einer Vorrichtung, basierend auf einer Zellkulturplatte (1), welche eine Vielzahl von auf der Oberseite der Zellkulturplatte (1) offenen und mit Böden (6) verschlossene Bohrungen (2) aufweist, in denen Zellen (8) aufgenommen werden, **dadurch gekennzeichnet, dass**
Nährstoffe mit oder ohne Sauerstoff besagte, als Zellkulturräume (2') fungierende und die Zellen (8) enthaltende Bohrungen (2) kontinuierlich oder diskontinuierlich durchströmen und durch diese geleitet werden.

2. Verfahren nach Anspruch 1, **dadurch gekennzeichnet, dass** der Sauerstoff durch eine gaspermeable Ausbildung der Böden (6) in Form einer gaspermeablen Membran oder Folie (9) in die Bohrungen (2) eingebracht wird.

3. Verfahren nach Anspruch 1 oder 2, **dadurch gekennzeichnet, dass** die Nährstoffe durch eine mikroporöse Ausbildung der Böden (6) in Form einer mikroporösen Membran oder Folie (9) in die Bohrungen (2) eingebracht werden.

4. Verfahren nach einem der Ansprüche 1 - 3, **dadurch gekennzeichnet, dass** die Zellkulturplatte (1) mit einer Deckplatte (10) abgedeckt ist.

5. Verfahren nach einem der Ansprüche 1 - 4, **dadurch gekennzeichnet, dass** die Zellkulturplatte (1) in eine Wanne (16) eingesetzt ist mit Öffnungen (18) zur Sauerstoffzufuhr.

6. Verfahren nach einem der Ansprüche 1 - 5, **dadurch gekennzeichnet, dass** in wenigstens einen Teil der Bohrungen (2) der Zellkulturplatte (1) Einsätze (3) einsetzbar sind, die jeweils mit Zulaufbohrungen (4) und Rücklaufbohrungen (5) versehen sind, durch welche Nährstoffe und Sauerstoff eingebracht und abgeführt werden können.

7. Verfahren nach Anspruch 6, **dadurch gekennzeichnet, dass** die Einsätze (3) jeweils auf ihrem zu den Böden (6) gerichteten Seiten mit einem Trennsteg (7) zwischen den Zulaufbohrungen (4) und den Rücklaufbohrungen (5) versehen sind, so dass die über die Zulaufbohrung (4) eingebrachten Nährstoffe nicht direkt zur Rücklaufbohrung (5) strömen und abgeführt werden können.

8. Verfahren nach einem der Ansprüche 1 - 5, **dadurch gekennzeichnet, dass** die Zellkulturplatte (1) mit einer Deckplatte (10) abgedeckt ist, welche Zulaufanschlüsse (4') und Rücklaufanschlüsse (5') aufweist, die in die Bohrungen (2) hineinragen und durch welche Nährstoffe und Sauerstoff eingebracht und abgeführt werden können.

9. Verfahren nach Anspruch 8, **dadurch gekennzeichnet, dass** die Zulaufanschlüsse (4') tiefer in die Bohrungen (7) hineinragen als die Rücklaufanschlüsse (5'), so dass die über die Zulaufanschlüsse (4') eingebrachten Nährstoffe nicht direkt zu den Rücklaufanschlüssen (5') strömen und abgeführt werden können.

10. Verfahren nach Anspruch 8 oder 9, **dadurch gekennzeichnet, dass** die Deckplatte (10) zweiteilig ausgebildet ist, mit einer oberen Abdeckung (10a) und einer unteren Abdeckung (10b), wobei die beiden Abdeckungen (10a, 10b) elastisch oder zueinander beweglich miteinander verbunden sind.

11. Verfahren nach Anspruch 10, **dadurch gekennzeichnet, dass** die untere Abdeckung (10b) mit Ansätzen (15) versehen ist, die in wenigstens einen Teil der Bohrungen (2) ragen, und durch welche die Zulaufanschlüssen (4') und Rücklaufanschlüssen (5') geschoben sind, die wiederum durch die obere Abdeckung (10a) geführt sind.

12. Verfahren nach Anspruch 11, **dadurch gekennzeichnet, dass** die Einheit, bestehend aus Zellkulturplatte (1), welche eine gaspermeable Ausbildung der Böden (6) in Form einer gaspermeablen Membran oder Folie (9) aufweist, oberer Abdeckung (10a), unterer Abdeckung (10b) und Ansätzen (15), in eine Wanne (16) gesetzt ist, wobei die Zellkulturplatte (1) umlaufend dicht auf der Wanne aufsitzt, und Sauerstoff durch die Bohrungen (18) in der Wanne durch die gaspermeable Membran oder Folie (9) in die Zellkulturräume (2') geführt wird.

13. Verfahren nach Anspruch 12, **dadurch gekennzeichnet, dass** sich zwischen Wanne (16) und Zellkulturplatte (1) ein Zwischenraum (17) befindet.

14. Verfahren nach einem der Ansprüche 1 - 13, **dadurch gekennzeichnet, dass** die Zufuhr und Abfuhr von Nährstoffen mit oder ohne Sauerstoff zu den einzelnen Zellkulturräumen (2') über einen gemeinsamen Zulaufanschluss (20), einen gemeinsamen Ablaufanschluss (23) sowie einzelnen Zweigleitungen (21),
die von dem Zulaufanschluss abzweigen und einzelnen Zweigleitungen (22), die von dem Ablaufanschluss abzweigen, und jeweils in den einzelnen Zellkulturräumen münden, erfolgt.

15. Verfahren nach Anspruch 14, **dadurch gekennzeichnet, dass** die Anschlüsse (20, 21, 22, 23) durch Leitungen, Aussparungen oder Kanäle in der Deckplatte (10) erfolgen.

16. Verfahren zum automatisierten Züchten und/oder Behandeln von Zellen für diagnostische Zwecke mittels einer Vorrichtung basierend auf einer Zellkulturplatte (1), welche eine Vielzahl von auf der Oberseite der Zellkulturplatte (1) offenen und mit Böden (6) verschlossene Bohrungen (2) aufweist, die als Zellkulturräume (2') fungieren und in denen Zellen (8) aufgenommen werden, **dadurch gekennzeichnet, dass** die Zellkulturräume (2') mit unterschiedlichen Drucken über Druckanschlüsse (27) beaufschlagt werden.

17. Verfahren nach Anspruch 16, **dadurch gekennzeichnet**, das der Druck auf die Zellkulturräume (2') durch Unterdrucksetzung von Zwischenräumen (28, 29) über und unterhalb der Zellkulturplatte (1), vermittelt wird über eine elastische als Boden (6) dienende Membran oder Folie (9) und eine elastische Membran oder Folie (26), mit welcher die Bohrung (2) auf ihrer Oberseite abgedeckt ist.

18. Verfahren nach Anspruch 17, **dadurch gekennzeichnet, dass** die Membran oder Folie (9) gaspermeable oder mikroporös ist.

19. Verfahren nach Anspruch 17 oder 18, **dadurch gekennzeichnet, dass** die Membran oder Folie (26) gaspermeable oder mikroporös ist.

20. Verfahren nach Anspruch 18 oder 19, **dadurch gekennzeichnet, dass** Nährstoffe mit oder ohne Sauerstoff besagte, als Zellkulturräume (2') fungierende und die Zellen (8) enthaltende Bohrungen (2) kontinuierlich oder diskontinuierlich durchströmen und durch diese geleitet werden.

21. Verfahren nach einem der Ansprüche 16 - 20, **dadurch gekennzeichnet, dass** in den Zellkulturräumen (2') rhythmische oder intermittierende Druckbelastungen erzeugt werden, so dass die Zellen (8) entsprechend mechanisch bewegt werden.

## Claims

1. Method for the automated cultivation and/or treatment of cells for diagnostic purposes by means of a device based on a cell culture plate (1) which has a multiplicity of holes (2) which are open on the top of the cell culture plate (1) and are sealed by bases (6) and in which cells (8) are accommodated, **characterized in that**
nutrients flow continuously or discontinuously with or without oxygen through said holes (2) functioning as cell culture spaces (2') and containing the cells (8) and are passed through them.

2. Method according to Claim 1, **characterized in that** the oxygen is introduced into the holes (2) through a gas-permeable embodiment of the bases (6) in the form of a gas-permeable membrane or film (9).

3. Method according to Claim 1 or 2, **characterized in that** the nutrients are introduced into the holes (2) through a microporous embodiment of the bases (6) in the form of a microporous membrane or film (9).

4. Method according to one of Claims 1 - 3, **characterized in that** the cell culture plate (1) is covered by a cover plate (10).

5. Method according to one of Claims 1 - 4, **characterized in that** the cell culture plate (1) is inserted into a tank (16) having apertures (18) for the supply of oxygen.

6. Method according to one of Claims 1 - 5, **characterized in that** inserts (3) which are each provided with feed holes (4) and return holes (5) through which nutrients and oxygen can be introduced and discharged can be inserted into at least some of the holes (2) of the cell culture plate (1).

7. Method according to Claim 6, **characterized in that** the inserts (3) are each provided on their sides facing the bases (6) with a dividing tab (7) between the feed holes (4) and the return holes (5), so that the nutrients introduced via the feed hole (4) cannot flow directly to the return hole (5) and be discharged.

8. Method according to one of Claims 1 - 5, **characterized in that** the cell culture plate (1) is covered by a cover plate (10) which has feed connections (4') and return connections (5') which project into the holes (2) and through which nutrients and oxygen can be introduced and discharged.

9. Method according to Claim 8, **characterized in that** the feed connections (4') project deeper into the holes (2) than the return connections (5'), so that the nutrients introduced via the feed connections (4') cannot flow directly to the return connections (5') and be discharged.

10. Method according to Claim 8 or 9, **characterized in that** the cover plate (10) has a two-part design, with an upper cover (10a) and a lower cover (10b), where the two covers (10a, 10b) are connected to one another in an elastic or mutually movable manner.

11. Method according to Claim 10, **characterized in that** the lower cover (10b) is provided with shoulders (15), which project into at least some of the holes (2) and through which are pushed the feed connections (4') and return connections (5'), which are in turn fed through the upper cover (10a).

12. Method according to Claim 11, **characterized in that** the unit consisting of cell culture plate (1) which has a gas-permeable embodiment of the bases (6) in the form of a gas-permeable membrane or film (9), upper cover (10a), lower cover (10b) and shoulders (15) is placed in a tank (16), where the cell culture plate (1) sits on the tank in a circumferentially leakproof manner, and oxygen is fed through the holes (18) in the tank through the gas-permeable membrane or film (9) into the cell culture spaces (2').

13. Method according to Claim 12, **characterized in that** an interspace (17) is located between the tank (16) and cell culture plate (1).

14. Method according to one of Claims 1 - 13, **characterized in that** the feed and discharge of nutrients with or without oxygen to or from the individual cell cultures space (2') takes place via a common feed connection (20), a common discharge connection (23) and individual branch lines (21) which branch off from the feed connection and individual branch lines (22) which branch off from the discharge connection and in each case run into the individual cell culture spaces.

15. Method according to Claim 14, **characterized in that** the connections (20, 21, 22, 23) take place through lines, recesses or channels in the cover plate (10).

16. Method for the automated cultivation and/or treatment of cells for diagnostic purposes by means of a device based on a cell culture plate (1), which has a multiplicity of holes (2) which are open on the top of the cell culture plate (1) and are sealed by bases (6) and which function as cell culture spaces (2') and in which cells (8) are accommodated, **characterized in that** the cell culture spaces (2') are pressurized with different pressures via pressure connections (27).

17. Method according to Claim 16, **characterized in that** the pressure on the cell culture spaces (2') is mediated by pressurization of interspaces (28, 29) above and below the cell culture plate (1) via an elastic membrane or film (9) serving as base (6) and an elastic membrane or film (26) by means of which the hole (2) is covered on its top.

18. Method according to Claim 17, **characterized in that** the membrane or film (9) is gas-permeable or microporous.

19. Method according to Claim 17 or 18, **characterized in that** the membrane or film (26) is gas-permeable or microporous.

20. Method according to Claim 18 or 19, **characterized in that** nutrients flow continuously or discontinuously with or without oxygen through said holes (2) functioning as cell culture spaces (2') and containing the cells (8) and are passed through them.

21. Method according to one of Claims 16 - 20, **characterized in that** rhythmic or intermittent pressure loads are generated in the cell culture spaces (2'), so that the cells (8) are moved mechanically in a corresponding manner.

## Revendications

1. Procédé pour la culture et/ou le traitement automatisés de cellules à des fins de diagnostic au moyen d'un dispositif sur la base d'une plaque de culture de cellules (1), laquelle comporte une multiplicité de trous (2) qui débouchent sur le sommet de la plaque de culture de cellules (1) et qui sont scellés par des bases (6) et dans lesquels des cellules (8) sont logées, **caractérisé en ce que**
des nutriments circulent de façon continue ou discontinue avec ou sans oxygène au travers desdits trous (2) fonctionnant en tant qu'espaces de culture de cellules (2') et contenant les cellules (8) et passent au travers.

2. Procédé selon la revendication 1, **caractérisé en ce que** l'oxygène est introduit dans les trous (2) au travers d'un mode de réalisation perméable aux gaz des bases (6) sous la forme d'une membrane ou d'un film perméable aux gaz (9).

3. Procédé selon la revendication 1 ou 2, **caractérisé en ce que** les nutriments sont introduits dans les trous (2) au travers d'un mode de réalisation microporeux des bases (6) sous la forme d'une membrane ou d'un film microporeux (9).

4. Procédé selon l'une des revendications 1 - 3, **caractérisé en ce que** la plaque de culture de cellules (1) est recouverte par une plaque de recouvrement (10).

5. Procédé selon l'une des revendications 1 - 4, **caractérisé en ce que** la plaque de culture de cellules (1) est insérée dans un réservoir (16) comportant des ouvertures (18) pour l'alimentation en oxygène.

6. Procédé selon l'une des revendications 1 - 5, **caractérisé en ce que** des inserts (3) qui sont chacun munis de trous d'alimentation (4) et de trous de retour (5) au travers desquels des nutriments et de l'oxygène peuvent être introduits et déchargés peuvent être insérés dans au moins certains des trous (2) de la plaque de culture de cellules (1).

7. Procédé selon la revendication 6, **caractérisé en ce que** les inserts (3) sont chacun munis sur leurs côtes faisant face aux bases (6) d'une patte de division (7) entre les trous d'alimentation (4) et les trous de retour (5), de telle sorte que les nutriments introduits via le trou d'alimentation (4) ne puissent pas circuler directement jusqu'au trou de retour (5) et être déchargés.

8. Procédé selon l'une des revendications 1 - 5, **caractérisé en ce que** la plaque de culture de cellules (1) est recouverte par une plaque de recouvrement (10) qui comporte des connexions d'alimentation (4') et des connexions de retour (5') qui font saillie dans les trous (2) et au travers desquelles des nutriments et de l'oxygène peuvent être introduits et déchargés.

9. Procédé selon la revendication 8, **caractérisé en ce que** les connexions d'alimentation (4') font saillie plus profondément dans les trous (2) que les connexions de retour (5'), de telle sorte que les nutriments introduits via les connexions d'alimentation (4') ne puissent pas circuler directement jusqu'aux connexions de retour (5') et être déchargés.

10. Procédé selon la revendication 8 ou 9, **caractérisé en ce que** la plaque de recouvrement (10) présente une conception en deux parties, avec un recouvrement supérieur (10a) et un recouvrement inférieur (10b), où les deux recouvrements (10a, 10b) sont connectés l'un à l'autre d'une manière élastique ou mobile de façon mutuelle.

11. Procédé selon la revendication 10, **caractérisé en ce que** le recouvrement inférieur (10b) est muni d'épaulements (15), qui font saillie dans au moins certains des trous (2) et au travers desquels les connexions d'alimentation (4') et les connexions de retour (5') sont poussées, lesquelles sont à leur tour acheminées au travers du recouvrement supérieur (10a).

12. Procédé selon la revendication 6, **caractérisé en ce que** l'unité constituée par une plaque de culture de cellules (1) qui comporte un mode de réalisation perméable aux gaz des bases (6) sous la forme d'une membrane ou d'un film perméable aux gaz (9), un recouvrement supérieur (10a), un recouvrement inférieur (10b) et des épaulements (15) est placée dans un réservoir (16), où la plaque de culture de cellules (1) repose sur le réservoir d'une manière étanche circonférentiellement, et de l'oxygène est alimenté au travers des trous (18) dans le réservoir au travers de la membrane ou du film perméable aux gaz (9) dans les espaces de culture de cellules (2').

13. Procédé selon la revendication 12, **caractérisé en ce qu'**un inter-espace (17) est localisé entre le réservoir (16) et la plaque de culture de cellules (1).

14. Procédé selon l'une des revendications 1 - 13, **caractérisé en ce que** l'alimentation et la décharge de nutriments avec ou sans oxygène sur ou depuis l'espace de culture de cellules individuel (2') sont réalisées via une connexion d'alimentation commune (20), une connexion de décharge commune (23) et des lignes de dérivation individuelles (21) qui sont dérivées depuis la connexion d'alimentation et des lignes de dérivation individuelles (22) qui sont dérivées depuis la connexion de décharge et qui, dans chaque cas, courent dans les espaces de culture de cellules individuels.

15. Procédé selon la revendication 14, **caractérisé en ce que** les connexions (20, 21, 22, 23) sont réalisées par l'intermédiaire de lignes, d'évidements ou de canaux dans la plaque de recouvrement (10).

16. Procédé pour la culture et/ou le traitement automatisés de cellules à des fins de diagnostic au moyen d'un dispositif sur la base d'une plaque de culture de cellules (1), laquelle comporte une multiplicité de trous (2) qui débouchent sur le sommet de la plaque de culture de cellules (1) et qui sont scellés par des bases (6) et qui fonctionnent en tant qu'espaces de culture de cellules (2') et dans lesquels des cellules (8) sont logées, **caractérisé en ce que** les espaces de culture de cellules (2') sont pressurisés selon différentes pressions via des connexions de pression (27).

17. Procédé selon la revendication 16, **caractérisé en ce que** la pression sur les espaces de culture de cellules (2') est facilitée par la pressurisation d'inter-espaces (28, 29) au-dessus et au-dessous de la plaque de culture de cellules (1) via une membrane ou un film élastique (9) jouant le rôle de base (6) et une membrane ou un film élastique (26) au moyen de laquelle/duquel le trou (2) est recouvert sur son sommet.

18. Procédé selon la revendication 17, **caractérisé en ce que** la membrane ou le film (9) est perméable aux gaz ou microporeuse/eux.

19. Procédé selon la revendication 17 ou 18, **caractérisé en ce que** la membrane ou le film (26) est perméable aux gaz ou microporeuse/eux.

20. Procédé selon la revendication 18 ou 19, **caractérisé en ce que** des nutriments circulent de façon continue ou discontinue avec ou sans oxygène au travers desdits trous (2) fonctionnant en tant qu'espaces de culture de cellules (2') et contenant les cellules (8) et passent au travers.

21. Procédé selon l'une des revendications 16 - 20, **caractérisé en ce que** des charges de pression rythmiques ou intermittentes sont générées dans les espaces de culture de cellules (2'), de telle sorte que les cellules (8) soient déplacées mécaniquement d'une manière correspondante.
